# EUROPEAN PATENT SPECIFICATION

(11) **EP 1 392 756 B1**
(45) Date of publication and mention of the grant of the patent: **14.02.2007**
(21) Application number: 02754661.3
(22) Date of filing: 03.06.2002
(51) Int. Cl.: C08G 69/10, A61K 9/127

(54) **LIPID-POLYMER-CONJUGATES**
LIPID-POLYMER KONJUGATEN
CONJUGUES LIPIDE-POLYMERE

(30) Priority: 01.06.2001 EP 01202107
(43) Date of publication of application: 03.03.2004
(73) Proprietor: Astellas Pharma Europe B.V., 2353 EW Leiderdorp (NL)
(72) Inventor: METSELAAR, J. M., Utrecht Inst. of Pharm. Sciences, 3508 TB Utrecht (NL); HENNINK, W. E., Utrecht Inst. of Pharm. Sciences, 3508 TB Utrecht (NL); DE VRINGER, Tom, c/o Yamanouchi Europe B.V., NL-2350 AC Leiderdorp (NL); DE BOER, L. W. T., c/o Yamanouchi Europe B.V., NL-2350 AC Leiderdorp (NL); OUSSOREN, C., c/o Utrecht Inst. of Pharm Sciences, 3508 TB Utrecht (NL); STORM, G., c/o Utrecht Inst. of Pharm Sciences, 3508 TB Utrecht (NL); BRUIN, P., 5503 CK Veldhoven (NL)
(86) International application number: PCT/EP2002/006432
(87) International publication number: WO 2002/098951

(56) References cited:
- WO-A-94/20073
- WO-A-99/61512
- ZHOU X ET AL: "LIPOPHILIC POLYLYSINES MEDIATE EFFICIENT DNA TRANSFECTION IN MAMMALIAN CELLS" BIOCHIMICA ET BIOPHYSICA ACTA. BIOMEMBRANES, AMSTERDAM, NL, vol. 1065, no. 1, 31 May 1991 (1991-05-31), pages 8-14, XP000197616 ISSN: 0005-2736
- ZHOU X. ET AL: "DNA transfection mediated by cationic liposomes containing lipopolylysine: characterization and mechanism of action" BIOCHIMICA ET BIOPHYSICA ACTA, vol. 1189, 1994, pages 195-203, XP001039665 Amsterdam, NL
- SHIBATA A ET AL: "PREPARATION AND CHARACTERIZATION OF LIPOSOMES INCORPORATING HYDROPHOBIC POLY(AMINO ACID)S WITH DIFFERENT SECONDARY STRUCTURE" CHEMICAL AND PHARMACEUTICAL BULLETIN, PHARMACEUTICAL SOCIETY OF JAPAN. TOKYO, JP, vol. 42, no. 5, 1 May 1994 (1994-05-01), pages 1151-1153, XP000466176 ISSN: 0009-2363

## Description

The present invention relates to lipid-polymer-conjugates, their preparation and their uses.

### BACKGROUND OF THE INVENTION

Lipid-polymer-conjugates are well-known and are used for a variety of different applications. One of these is the inclusion into colloidal carrier compositions, such as vesicular bilayer systems, such as liposomes, niosomes and reversed vesicles, micellar systems, nanocapsules, nanospheres etc.. A well-known representative of such colloidal carrier compositions is formed by liposomes. Although hereinafter especially liposomes are mentioned, the reader should bear in mind that the discussions, disclosures and teachings relate to other colloidal carrier compositions as well.

Liposomes, which belong to the group of colloidal carrier particles, are small vesicles consisting of one or more concentric lipid bilayers enclosing an aqueous space. Because of their structural versatility in terms of size, surface charge, lipid composition, bilayer fluidity and because of their ability to encapsulate almost every drug, their importance as drug delivery systems was readily appreciated. However, on intravenous injecting of liposomes, these are recognised as foreign particles by the Mononuclear Phagocyte System (MPS) and rapidly cleared from the circulation to organs rich in phagocytic cells, like liver, spleen and bone marrow. Several possibilities to reduce this effect have been identified, such as decreasing the particle size of the liposomes and changing the surface charge of the liposomes. Another development relates to surface modification of the liposomes by the introduction of specific hydrophilic polymeric components on the liposomal surface, which components reduce protein adsorption on the particle surface. Consequently such liposomes are protected against recognition by cells of the MPS and have a prolonged residence time in the general circulation. A well-known example of modification of the liposomal surface is the incorporation during the preparation of liposomal compositions of a lipid derivative of the hydrophilic polymer polyethylene glycol (PEG). Usually this polymer is terminus-modified with a hydrophobic moiety, which is the residue of a phosphatidyl ethanolamine derivative or a long-chain fatty acid. Polyethylene glycol per se is a rather stable polymer, which is a repellant of protein adhesion and which is not subject to enzymatic or hydrolytic degradation under physiological conditions. Good results with respect to extending plasma half life and diminishing accumulation into the organs rich in phagocytic cells have been obtained following intravenous administration of liposomes, having a PEG-grafted surface, to various animal species and also to human beings (Storm G., Belliot S.O., Daemen T. and Lasic D.D.: Surface modification of nanoparticles to oppose uptake by the mononuclear phagocyte system in Adv. Drug Delivery Rev. 17, 31-48, (1995); Moghimi S.M., Hunter A.C. and Murray J.C.: Long-circulating and target-specific nanoparticles; theory to practice in Pharmacol. Rev. 53, 283-318, (2001); Boerman O.C., Dams E.T., Oyen W.J.G., Corstens F.H.M. and Storm G.: Radiopharmaceuticals for scintigraphic imaging of infection and inflammation in Inflamm. Res. 50, 55-64, (2001)). Marketing approvals for such liposomal preparations, containing doxorubicine, have been obtained.

Meanwhile several disadvantages of the use of the polymer polyethylene glycol in long-circulating liposomes have been encountered. The accumulation of PEG-grafted liposomes in macrophages and the skin is of some concern due to non-biodegradability. Loss of the long-circulation property (fast clearance) on injecting PEG-liposomes for a second time has been observed (Dams E.T., Laverman P., Oijen W.J., Storm G., Scherphof G.L., Van der Meer J.W., Corstens F.H. and Boerman O.C.: Accelerated blood clearance and altered biodistribution of repeated injections of sterically stabilized liposomes in J. Pharmacol. Exp. Ther. 292. 1071-1079, (2000)). Recent studies with PEG-liposomes in patients have shown that PEG-liposomes can induce acute side effects (facial flushing, tightness of the chest, shortness of breath, changes in blood pressure), which resolve immediately when the administration (infusion) of the PEG-liposome formulation is terminated. Recent data point to a role of complement activation in the induction of side effects (Szebeni J., Baranyi L., Savay S., Lutz H., Jelezarova E., Bunger R. and Alving C.R.: The role of complement activation in hypersensitivity to Pegylated liposomal doxorubicin (Doxil) in J. Liposome Res. 10, 467-481, (2000)). Until now the commercially available preparations based on PEG-liposomes are aqueous suspension preparations. It is well-known that the shelf life of liposomal aqueous suspension preparations in general and also of PEG-liposomes is rather limited. Several techniques how to remove the vehicle or continuous phase of such preparations are known, such as, spray-drying, diafiltration, rotational evaporation etc., and preferably freeze-drying. Recently a freeze-drying method, which improved the long term shelf life of PEG-liposomes, containing the technetium-chelator hydrazino nicotinamide, was proposed (Laverman P., van Bloois L., Boerman O.C., Oyen W.J.G., Corstens F.H.M. and Storm G.: Lyophilisation of Tc-99m-HYNIC labelled PEG-liposomes in J. Liposome Res. 10(2&3), page 117-129 (2000)), but further investigations into the results and applicability of this technique to liposomal preparations are required.

The disadvantages inherent to the use of polyethylene glycol urged investigators to look for alternative polymers. Many polymers have been suggested as suitable candidates for derivatising them with (vesicle-forming) lipids for incorporation into liposomes (see e.g. EP-0688207). The hydrophilic water soluble polymers poly(vinylpyrrolidone), poly(acryloylmorpholine), poly(2-(m)ethyl-2-oxazoline, polyacrylamide and polyglycerol have shown to prolong the circulation time of liposomes after intravenous administration to a certain extent. However, until now such lipid-polymer conjugates have not been applied in commercially available drug preparations, mainly because they have not shown any advantages over the known lipid-PEG-conjugates. Therefore there still is a need to find a polymer, which can be derivatised with a lipid to enable incorporation into colloidal carrier compositions, such as liposomes, such polymer having long-circulating properties and in addition thereto having advantages over PEG, such as biodegradability.

### SUMMARY OF THE INVENTION

Lipid-polymer conjugates are provided, which are obtainable from an amphiphilic lipid, consisting of at least one hydrophobic apolar moiety and a hydrophilic polar head group, and a polymer or a monomeric precursor therefor, having an N- and a C-terminal end group, wherein the polymer is a poly-(amino acid), a poly-(amino acid derivative) or a poly-(amino acid analogue) and wherein the lipid is covalently attached to the N or C terminal end group of the polymer, the polymer having the following formula:
-[NHCHR(CH₂)ₘCO]ₙ-, wherein -R is defined as: -H , -CH₃ , -CHCH₃OR₁, -(CH₂)ₓOR₁,-(CH₂)ₓ-CO-NHR₁, -(CH₂)ₓ-NH-CO-R₁, -(CH₂)ₓ-SO_{y}CH₃, or -(CH₂)ₓCOOH; -R₁ is H or (C₁-C₄)alkyl, substituted with one or more hydroxy groups or one di(C₁-C₄)alkylamine group; x is 0-4; m = 1 or 0 and y = 1 or 2. Also provided are methods to prepare such conjugates and uses of the conjugates.

### LEGENDS TO THE FIGURES

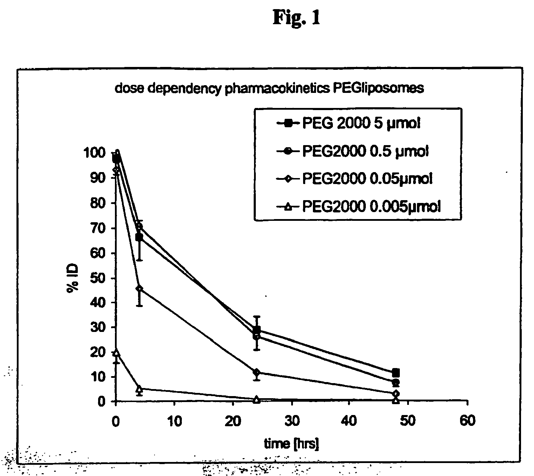

Figure 1 is a graphical representation of the mean values for the calculated percentage injected dose in blood samples versus time for PEG-DSPE-containing liposomal preparations, having a different amount of Total Lipid (example 22).

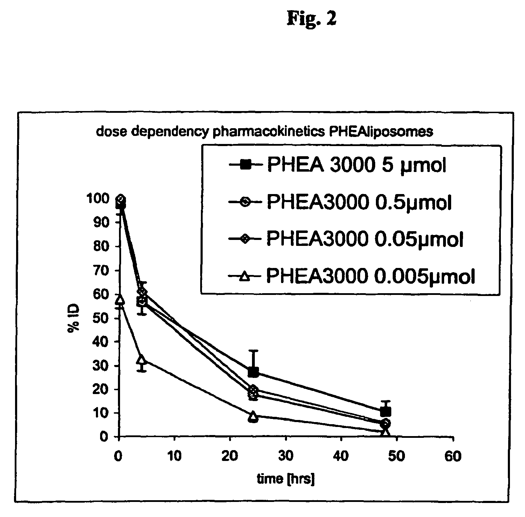

Figure 2 is a graphical representation of the mean values for the calculated percentage injected dose in blood samples versus time for PHEA-DODASuc-containing liposomal preparations, having a different amount of Total Lipid (example 22).
Figure 3 is a graphical representation of the percentage encapsulated prednisolone phosphate in PEG-DSPE and PHEA-DODASuc, respectively, containing liposomal preparations versus time (example 23).
Figure 4 is a graphical representation of the concentration of prednisolone phosphate encapsulated in PEG-DSPE and PHEA-DODASuc containing liposomes in blood versus time (example 24).
Figure 5 is a graphical representation of the paw inflammation score versus time before and after a single intravenous injection of saline and prednisolone phosphate-containing liposomes (coated with PEG-DSPE and PHEA-DODASuc respectively) (example 24).
Figure 6 is a graphical representation of the percentage injected dose of liposomes found in liver, spleen and liver+spleen after intravenous administration of liposomes, containing as the lipid-polymer-conjugates PEG-DSPE, PHEG-diaminobutane DODASuc, PHPG diaminobutane DODASuc, PHBG diaminobutane DODASuc and PHEA-DODASuc respectively, and conventional liposomes (BARE) (Example 21).

### DETAILED DESCRIPTION OF THE INVENTION

The amphiphilic lipid-polymer-conjugates of the present invention are obtainable from an amphiphilic lipid and a polymer or a monomeric precursor therefor.

The amphiphilic lipids to be used in the lipid-polymer conjugate according to the invention may be selected from a variety of synthetic or naturally occurring lipids, consisting of at least one hydrophobic apolar tail and a hydrophilic polar head group, such as vesicle-forming lipids and membrane lipids.
An important feature of the amphiphilic lipid to be used in the lipid-polymer conjugate is that the lipid contains a functional group at its polar head group suitable for covalent attachment to a polymer chain. The polar head group is for example a primary or secondary amine group, a hydroxyl group, an aldehyde group, a halide or a carboxylic acid group. The hydrophobic moiety of the lipid enables the incorporation of the lipid-polymer conjugates into bilayer structures, such as liposomes and acts as an anchor.
Examples of amphiphilic lipids are phospholipids, glycolipids, ceramides, cholesterol and derivatives, saturated or partially unsaturated, branched or straight-chain C₈-C₅₀ mono- or di-alkylamine, arylalkylamines, cycloalkylalkylamines, alkanols, aldehydes, carbohalides or alkanoic acids and the anhydrides thereof, wherein the total number of C-atoms preferably is 25 or higher.
More specifcally, examples of suitable amphiphilic lipids are phosphatidyl choline, phosphatidyl ethanolamine, phosphatidyl inositol, sphingomyeline, stearylamine, myristylalcohol, cholesterol and palmitic acid.
A preferred amphiphilic lipid in the lipid-polymer-conjugate is a lipid having two hydrophobic chains, typically alkyl chains, and a polar head group, containing a functional group, as described above. Phosphatidyl ethanolamine derivatives and in particular distearyl phosphatidyl ethanolamine, are such preferred phospholipids since they contain a reactive amino group.
Further preferred amphiphilic lipids have as the hydrophilic polar head group a primary or secondary amine and two saturated or unsaturated C₈-C₅₀ branched or straight chain hydrophobic apolar moieties. Examples thereof are 1-heptadecyloctadecylamine and distearylamine-containing compounds, such as distearylamine and N-succinyl-dioctadecylamine (DODASuc).

The polymer part of the lipid-polymer-conjugates of the present invention is formed by a poly-(amino acid), a poly-(amino acid derivative) or a poly-(amino acid analogue). A poly-(amino acid derivative) is a polymer, which consists of amino acid monomers, to which one or more substituents are attached. An example thereof is poly(2-hydroxyethyl)-L-glutamine. A poly-(amino acid analogue) as herein disclosed is a polymer, wherein the carbon atom chain length of the amino acid monomers is reduced or prolonged. Examples thereof are poly(-homoserine) and poly(pentahomoserine).
The polymer is a homo-polymer, consisting of monomers that are the same throughout the polymer chain, It is also possible that the polymer part consists of block co-polymers selected from the group consisting of poly-(amino acid), poly-(amino acid derivative) and poly-(amino acid analogue) or that the polymer part is formed by a series of alternating monomers or a controlled order of monomers or by random polymerisation of suitable monomers selected from the group consisting of one or more amino acids, amino acid derivatives and amino acid analogues. The polymers may be linear or branched and include graft polymers, but preferably are linear.
Useful amino acids are the naturally occurring α-amino acids. However also β-amino acids as well as nonprotein or non-naturally occurring amino acids have appeared to be of interest.

Both the L- and the D-configuration of the amino acids and derivatives can be used. When the amino acid sequence of the polymer in the lipid-polymer-conjugate is formed by residues of the L-amino acid, the resulting polymer will be subject to enzymatic degradation. On the other hand, when the amino acid sequence of the polymer in the lipid-polymer-conjugate of this invention is formed by the D-amino acid, the resulting polymer is likely to be stable towards peptide-degrading enzymes. Also mixtures of the L- and D-amino acids can be used. Taking into account the different properties of the polymers, surface-modification of colloidal carrier particles, in which the lipid-polymer-conjugates of the invention are incorporated, can be adjusted by selective use of the L- and/or D-form of the starting materials for preparation of the conjugates.
An important property of the poly-(amino acid), poly-(amino acid derivative) and poly-(amino acid analogue) compounds, which are suitable for incorporation into the lipid-polymer-conjugates of the present invention, is that they are soluble in water (at least 1 part in 100 parts of water, preferably 1 part in 30 parts of water and most preferably 1 part in 10 parts of water or less). The polymers can also be characterised by their χ**-**parameter in water. This polymer-solvent interaction parameter can be determined by e.g. membrane-osmometry. The polymers which can be advantageously used in the lipid-polymer conjugates according to this invention have a χ-parameter of ≤0.65, preferably ≤ 0.5 in water.
A further important feature of the polymers is that they contain no substantial amount of charged groups within a (physiological) pH-range of 4-8. Preferably neutral amino acid monomers or amino acid analogue monomers are used in the preparation of the polymers or amino acid derivative monomers, which are neutral or have been neutralised. As it appeared charged groups can be allowed to be present in a low percentage without disturbing the long-circulating properties of the colloidal carrier compositions according to the present invention. As has been demonstrated for co-polymers of 2-hydroxyethyl-L-glutamine and charged monomers, positively charged groups can be allowed to be present in a larger percentage than negatively charged groups.
Suitable monomers for the preparation of the polymer are amongst others alanine, threonine, valine, sarcosine, α-aminoadipic acid, α,γ-diaminobutyric acid derivatives, ornithine, glutamine and derivatives, including glutamic acid, asparagine and derivatives, including aspartic acid, lysine derivatives, methionine and derivatives, serine, its derivatives and analogues with additional CH₂-groups, such as homoserine and pentahomoserine. Suitable side-groups include the (C₁-C₄)-alkyl, hydroxyalkyl, dihydroxyalkyl, carbamoyl and aryl groups or combinations thereof, provided that the polymer remains water soluble. Examples of these groups are 2-hydroxyethyl, 3-hydroxypropyl, 4-hydroxybutyl and 2,3-dihydroxypropyl. Polymers which can be used are e.g. poly(D,L-serine) (PDLS), poly(2-hydroxyethyl)-D,L-glutamine (PDLHEG), poly(2-hydroxybutyl)-L-glutamine (PHBG) and the copolymer poly(HEG-co-glutamic acid) 1% glutamic acid (PHEG1%GA). Preferred polymers are poly(D,L-glutamine) (PDLG), poly(D,L-asparagine) (PDLA), poly(hydroxypropyl)-L-glutamine (PHPG), poly(2-hydroxypropyl)-L-glutamine (P2HPG) and the copolymers of beta-alanine and 2-hydroxyethyl-L-glutamine (PbAHEG), poly(HEG-co-dimethylaminoethyl-glutamine) containing 5 and 1% dimethylaminoethyl side groups (PHEG5%DG and PHEG1%DG). More preferred polymers are the homopolymers poly-[N-(2-hydroxyethyl)-L-glutamine] (PHEG), poly(2-hydroxyethyl)-L-asparagine (PHEA) and poly(D,L-methioninesulfoxide) (PDLMS).
The polymer chain contains between 5 and 500 monomer subunits, preferably between 20 and 100. The mean molecular weight of the polymer varies from 500 to 75,000, preferably from 2,000 to 15,000. The mean molecular weight can be assessed in different ways as known in the art. In the examples of the present application an estimate of the molecular weight has been made based on NMR-data.
For the preparation of the lipid-polymer-conjugates of the present invention manufacturing methods have preferably been used, wherein reactive groups in the side chains of the amino acid monomers were protected prior to polymerisation and coupling of the lipid.

The lipid-polymer-conjugates can be prepared according to methods known in the art. A well-known method to prepare polymers of amino acids involves the ring opening polymerisation of the corresponding amino acid N-carboxy-anhydride (NCA)s, optionally provided with one or more protective groups, initiated by nucleophiles such as (C₁-C₄) alkyl primary amines. Another method to obtain the lipid-polymer-conjugates comprises the use of an amine with a protected functional group, for instance N-Boc-1,4-diaminobutane, as the initiator in the ring opening NCA polymerisation. Although two extra steps, namely deprotection of the functional group and subsequently coupling to a lipid with a reactive group, are required in this process, the lipid-polymer-conjugates prepared by this method are also suitable for incorporating into the colloidal carrier compositions of this invention.
If the amphiphilic lipid is a C₈-C₅₀ branched or straight-chain mono- or di-alkyl, - hydroxyalkyl or -alkylene amine, an alkanol or a ceramide, this can be advantageously used as the initiator in the ring opening polymerisation process. This means that during the polymerisation the amphiphilic lipid is coupled to the polymer in one step. The molecular weight of the poly-amino acids strongly depends on the solvent or the combination of solvents, on the purity of the chemicals used and on the ratio of monomer/polymerisation initiator. Generally speaking, the higher the ratio monomer/polymerisation initiator, the higher the molecular weight of the polymer will be.
When a polymer of pre-defined composition should be prepared, the solid phase peptide synthesis method is preferably used.
Protective groups present in the repeating units of the polymer can be removed by aminolysis using an amino-alcohol such as 2-aminoetbanol, 3-aminopropanol or 2,3-dihydroxypropylamine.

The lipid-polymer-conjugates of the present invention can be advantageously incorporated into colloidal carrier compositions of the invention, such as vesicular bilayer systems, such as liposomes, niosomes and reversed vesicles, micellar systems, nanocapsules, nanospheres etc.. Preferred colloidal carrier systems are the vesicular bilayer systems.
On preparing liposomes the lipid-polymer-conjugate according to the invention is mixed with components, normally used in the preparation of liposomes, such as vesicle-forming lipids, stabilisers etc.. The conjugate is included at a molar concentration sufficient to extend the blood circulation time of the liposomes several fold over that of corresponding liposomes lacking the Lipid-polymer-conjugate. The polymer conjugate is typically included at 1-15 mole percent, preferably at 3-10 mole percent and most preferably at 5-7.5 mole percent
The average size of the liposomes, to be determined by Dynamic Light Scattering (DLS) techniques, is below 200 nm, preferably below 150 nm and most preferably below 100 nm. The lower limit for this type of colloidal carrier particles is 20 nm.
The Lipid-polymer-conjugates, when incorporated into charged liposomes, showed the ability to reduce the zeta-potential, thus demonstrating that the polymer grafting shielded the surface charge.
The compositions can be administered in several ways, but parenteral administration is preferred. Dependent on the active ingredient and on the medical indication or disorder to be treated, administration can be done by intravenous, subcutaneous, intramuscular, intraperitoneal, intra-articular etc. injection.
After intravenous administration of liposomal preparations in accordance with the present invention to rats it has been shown that the blood circulation time of the liposomes can be varied in accordance with the desired purpose. The blood circulation time is dependent on the lipid-polymer-conjugate used, in particular on the choice of the lipid/polymer combination, the molecular weight of the polymer and the grafting density. Results similar to those obtained with the corresponding PEG-grafted liposomes have been observed e.g. for lipid-PHEG-conjugates, lipid-PHEA-conjugates and lipid-PDLMS-conjugates, wherein the amphiphilic lipid contains a double hydrophobic tail (PHEG-diaminobutane DODASuc, PHEA-DODASuc and PDLMS-DODASuc).

The stability of liposomal preparations, prepared with the lipid-polymer-conjugates in accordance with the present invention, is generally improved as compared to that of conventional liposomal preparations. In addition thereto the stability of the liposomal preparations can be further improved by the proper selection of the lipid-polymer-conjugate. It will be appreciated that this selection is also dependent on the choice of the active agent. E.g. encapsulation of a water soluble derivative of a corticosteroid instead of the corticosteroid per se into a liposomal preparation will result in an increased stability of the liposomal preparation. Encapsulation of prednisolone phosphate into a polyhydroxyethylasparagine-DODASuc-conjugate-containing liposome gave a slightly better result than incorporation into a poly(2-hydroxyethyl)-L-glutamine-diaminobutane DODASuc-conjugate-containing liposome. A further improvement of the stability can be reached by removing the aqueous vehicle from the liposomal composition by methods well-known in the art, such as spray-drying, freeze-drying, rotational evaporation etc..

The lipid-polymer conjugates, incorporated into the colloidal carrier compositions according to the present invention, provide long-circulating properties to these compositions.

Under long-circulating properties is to be understood an increase in blood circulation time of the colloidal carrier composition, as compared with such composition, not containing the lipid-polymer-conjugate. The long-circulating properties can be determined according to methods known in the art (Torchilin VP, Shtilman MI, Trubetskoy VS, Whiteman K, Milstein AM.: Amphiphilic vinyl polymers effectively prolong liposome circulation time in vivo. Biochimica et Biophysica Acta (1994) 1195: 181-184; Torchilin VP, Trubetskoy VS, Whiteman KR, Caliceti P, Ferruti P, Veronese FM.: New synthetic amphiphilic polymers for steric protection of liposomes in vivo. Journal of pharmaceutical sciences (1995) 84 (9): 1049-1053). For liposomes a method has been provided in the examples. Therefore, such compositions and especially the vesicular ones, can be used for a variety of applications. Except as a circulating drug reservoir, they can be used for passive targeting to sites of pathology (tumours, infection, inflammation) and for active targeting to cells in the bloodstream, to endothelium (e.g. to angiogenesis-related receptors), e.g. by coupling to homing devices, such as monoclonal antibodies. Further applications may be an artificial oxygen delivery system, blood-pool imaging and an anti-fouling coating for biomaterials, such as catheters and blood vessel protheses.
In addition thereto the lipid-polymer-conjugates are biodegradable and therefore provide a lot of advantages, in particular due to the fact that there is no risk of accumulation in cells of the human or animal body.
Further the lipid-polymer-conjugates have shown that there is a reduced lipid-dose dependency as compared with PEG-liposomes.
Another additional, but very important advantage may be that an increased clearance after second injection of the compositions according to the invention is not always observed and
that the reduction in blood circulation time is moderate. This would mean a significant advantage as compared to colloidal carrier compositions, coated with PEG.

The colloidal carrier compositions according to the present invention provide a variety of possibilities for use in therapy, diagnosis, prophylaxis etc.. Due to the versatility of the lipid-polymer-conjugates, the components of which can be selected in accordance with the purpose, and the variety of colloidal carrier systems from which one can choose, it will be readily apparent that in general it will appear possible for every active agent to design an appropriate colloidal carrier composition. If in first instance after intravenous administration of compositions according to the invention no or only a slight effect on the blood circulation time is observed, the person skilled in the art can vary a lot of different parameters in the lipid-polymer-conjugate (e.g. molecular weight, drafting density, polymer, lipid etc.) and in the composition of the colloidal carrier to increase the circulation time according to the standard set. A very interesting effect is seen when compositions according to the invention contain a water soluble corticosteroid as the active agent. In an in vivo experimental arthritis model one single intravenous injection of such composition has appeared to be as effective as repeated injections of the non-encapsulated corticosteroid compound or when encapsulated in conventional liposomes. Interesting water soluble corticosteroids are budesonide phosphate and water soluble derivatives of flunisolide and fluticasone propionate. The favourable effects may be a complete and long-lasting remission of arthritis-associated symptoms, whilst the side-effects associated with corticosteroid-based therapy will be reduced, due to a reduction in the amount of corticosteroids that has to be administered and because corticosteroids, which normally show a fast clearance from the blood, can now be used. Also in other diseases, in which corticosteroids are the drugs of choice or are used as co-therapy, the beneficial effects of the compositions according to the present invention will be readily recognised. However, also other active agents show interesting effects in the compositions of the invention.

The following examples further illustrate the invention.

### EXAMPLES

### Example 1

### Poly(γ-benzyl-L-glutamate) having a heptadecyloctadecylamine end group (PBLG-heptadecyloctadecylamine)

0.94 g (3.6 mmol) γ-benzyl-L-glutamate N-carboxyanhydride (NCA) was dissolved in 5 ml dry DMF under a nitrogen atmosphere. A solution of 25 mg (0.05 mmol) 1-heptadecyloctadecylamine (Sigma Aldrich) in 1 ml dry chloroform was added at once. Almost immediately gas bubbles (CO₂) were formed. The solution was stirred for 1 day at room temperature and then precipitated into a 10-20 fold excess of water. The white precipitate was collected and dried in vacuo. Yield: 0.75 g.
Characterization:
¹H-NMR in CDCl₃ (δ in ppm relative to solvent peak):
   Benzylglutamate: 7.2 (C*₆H₅*), 5.0 (benzylic C*H*₂), 3.9 (α-C*H*), 2.8 & 2.2 (β & γ *CH*₂*),*
   Alkyl chain: 1.2 *(CH₂* alkyl chain), 0.85 (*CH*₃)

### Example 2

### Poly(N-(2-hydroxyethyl)-L-glutamine) having a heptadecyloctadecylamine end group (PHEG-heptadecyloctadecylamine)

To a solution of 0.60 g PBLG-heptadecyloctadecylamine (see above) in 5 ml dry DMF was added 0.15 g 2-hydroxypyridine and 0.8 ml ethanolamine. This solution was stirred for 3 days at room temperature under a nitrogen atmosphere. The solution was precipitated into a 10-20 fold excess of diethylether. The product was collected and dried in vacuo. The watersoluble polymeric product was dialysed against water in cellulose ester dialysis tubes (MWCO 500) for 2 days. Purified PHEG having the heptadecyloctadecyl end group was obtained after freeze-drying. Yield: 0.30 g.
Characterization:
¹H-NMR in DMSO-d6 (δ in ppm relative to solvent peak):
   Hydroxyethylglutamine: 4.1 (α-C*H*), 2.2 & 1.8-1.9 (β & γ C*H*₂), 3.4 (C*H*₂*-*OH), 3.1 (C*H*₂-NH₂),
   Alkyl chain: 1.2 (C*H*₂ alkyl chain), 0.8 (C*H*₃).
From the ratio of integrals of the stearyl signals and the α-CH signal the PHEG molecular weight was estimated to be 12,000.

### Example 3

### Poly-[N-(2-hydroxyethyl)-L-glutamine] having a distearylamine end group introduced via N-Boc-1,4-diaminobutane as initiator (PHEG-diaminobutane-DODASuc)

2.5 g (9.5 mmol) γ-benzyl-L-glutamate N-carboxyanhydride was dissolved in a mixture of 2.5 ml of dry ethylacetate and 12.5 ml of dry dichloromethane, and 0.95 ml (0.95 mmol) of a 1 molar solution of N-Boc-1,4-diaminobutane in dichloromethane as initiator was added. The mixture was stirred for 3 days under nitrogen at room temperature and then precipitated in methanol. Yield: 1.48 g PBLG-N-Boc-diaminobutane.
The ¹H-NMR (CDCl₃) spectrum showed N-Boc-l,4-diaminobutane signals at δ 1.4-1.3.
Maldi TOF ms:
Mass of the repeating benzylglutamic acid unit: n x 219.
*m*/*z* 1417(n=5), 1636 (n=6), 1855 (n=7), 2074 (n=8), corresponding to the masses of the sodium adduct with an N-Boc-1,4-diaminobutane (187 Da) group and a cyclic peptide end group (112 Da).
*m*/*z* 1433(n=5), 1652 (n=6), 1872 (n=7) corresponding to the masses of the potassium adduct with an N-Boc-1,4-diaminobutane (187 Da) group and a cyclic peptide end group (112 Da).

Deprotection of PBLG-N-Boc-diaminobutane.
738 mg of PBLG-N-Boc-diaminobutane was stirred during 3.5 hours in 5 ml of a solution of 4N HCl in dioxane. Subsequently the reaction mixture was evaporated on a Rotavap. The residue was dissolved in 5 ml of tetrahydrofuran and added dropwise to 80 ml of a NaHCO₃ solution (6.5 g in water). The product was filtered off, washed with water and dried in vacuum. Yield: 677 mg PBLG-diaminobutane.
¹H-NMR (CDCl₃) showed that the protective group was successfully removed.
Maldi TOF mass analysis showed the desired molmasses.
Mass of the repeating benzylglutamic acid unit: n x 219.
*m*/*z* 1318(n=5), 1537 (n=6), 1756 (n=7), 1976 (n=8), corresponding to the masses of the sodium adduct with a 1,4-diaminobutane (87 Da) group and a cyclic peptide end group (112 Da).

Coupling to DODASuc:
62 mg (0.1 mmol) of N-succinyl-dioctadecylamine (DODASuc, Schmitt et al, J. Am. Chem. Soc.1994, 116, 19, 8485-8491), 13.7 mg (0.12 mmol) of N-hydroxysuccinimide and 0.66 mg of dimethylaminopyridine (DMAP) were dissolved in 2 ml of dichlommethane. After cooling to 0 °C 24.6 mg (0.12 mmol) of N,N'-dicycohexylcarbodiimide (DCC) was added. The solution was stirred for 1 hour at 0 °C and overnight at room temperature. Then the insoluble dicyclohexylurea was filtered off, and the filtrate was added to a solution of 200 mg PBLG-diaminobutane in 3 ml of dichloromethane and 14 µl of triethylamine. After stirring overnight at room temperature the solution was added dropwise to methanol, filtrated and dried. Yield: 135 mg. The ¹H-NMR (CDCl₃) spectrum showed that distearyl was present, C*H*₂ signals at δ 1.4-1.2 and C*H*₃ signals at δ 0.9-0.8.
Maldi TOF mass analysis showed the desired molmasses indicating that DODASuc was coupled to PBLG-diaminobutane.
*m*/*z* 1922 (n=5), 2141 (n=6), 2360 (n=7), 2580 (n=8), 2799 (n=9), 3018 (n=10). corresponding to the masses of the sodium adduct of PBLG-diaminobutane-DODASuc, with a cyclic peptide end group (112 Da).

### Structure:

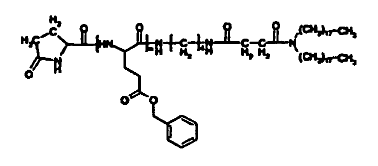

Aminolysis.
120 mg of PBLG-diaminobutane DODASuc and 10.8 mg of 2-HP were dissolved in 1.3 ml of dimethylformamide and 0.68 ml of 2-aminoethanol was added. After stirring for 24 hours at 40 °C under nitrogen the solution was added dropwise to chloroform. The product was filtered off and dried in vacuum. Yield: 83 mg PHEG-diaminobutane-DODASuc.
¹H-NMR (DMSO) showed distearyl signals at δ 1.2-1.4 (C*H*₂) and δ 0.8-0.9 (C*H*₃).
From the ratio of integrals of the stearyl signals and the α-CH signal the PHEG molecular weight was estimated to be 4,000.

### Structure:

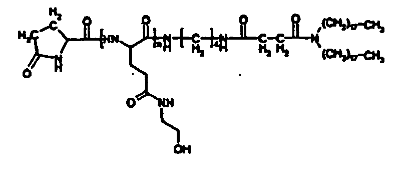

### Example 4

### Poly-[N-(2-hydroxyethyl)-L-glutamine] having an amine end group introduced by distearylamine as initiator (PHEG-distearylamine)

1.0 g (3.8 mmol) γ- benzyl-L-glutamate N-carboxyanhydride was dissolved in a mixture of 1 ml dry ethylacetate and 5 ml of dry chloroform. Subsequently 2 ml (0.19 mmol) of a solution of 163 mg distearylamine in 3.26 ml of chloroform was added. The flask was equipped with a CaCl₂ tube and the mixture was stirred under nitrogen during 4 days at room temperature. The mixture was added dropwise to methanol, the polymer was isolated by filtration and dried in vacuo. Yield: 757 mg PBLG-distearylamine.

### Reaction:

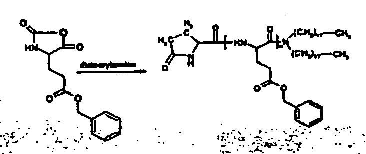

¹H-NMR (CDCl₃) analysis showed the distearyl signals in the product: CH₃ signal at δ 0.9 (t) and CH₂ signal at δ 1.3-1.2.
Maldi TOF ms:
Mass of the repeating benzylglutamic acid unit: n x 219.
*m*/*z* 1971 (n=6), 2190 (n=7), 2410 (n=8), 2629 (n=9), 2848 (n=10) corresponding to the masses of the sodium adduct with a distearylamine (521 Da) group and a cyclic peptide end group (112 Da).
*m*/*z* 2206 (n=7), 2427 (n=8), corresponding to the masses of the potassium adduct with a distearylamine (521 Da) group and a cyclic peptide end group (112 Da).

### Aminolysis

Aminolysis of the above prepared PBLG-distearylamine (600 mg) with aminoethanol and 2-hydroxypyridine as catalyst in dimethylformamide gave 430 mg of PHEG-distearylamine.
¹H-NMR (DMSO-d6) showed the distearylamine signals.

### Structure:

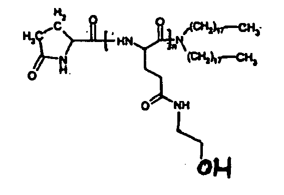

### Examples 5

### Poly-[N-(hydroxyalkyl)-L-glutamine] having a diaminobutane DODASuc endgroup PBLG-diaminobutaneBOC:

To a solution of 3 g benzyl-L-γ-glutamate N-carboxyanhydride (NCA) in 8 ml dry DMF was added a solution of 0.1 g N-BOC-1,4-diaminobutane in 1 ml of chloroform. Formation of gas (carbon dioxide) was noticed during the first hours. This solution was stirred for 1 day under a nitrogen atmosphere at room temperature. After precipitation into ca. 100 ml methanol the polymer was filtered off and dried, yielding 2 g PBLG containing a BOC-protected amino group.
¹H-NMR (CDCl₃) (δ relative to solvent peak):
BOC: 1.4 (CH₃)
PBLG: 2.2 & 2.6 (β,γ-CH₂), 4.0 (α-CH), 5.0 (benzyl CH₂), 7.3 (phenyl)

PBLG-diaminobutane (removal protective BOC group):
A solution of 1.1 g PBLG-diaminobutaneBOC in 5 ml 4N HCl/dioxane was stirred for 3-4 hours and then added dropwise to ca. 80 ml NaHCO₃ solution (6.5 g in water). The product was filtered off, washed with water and dried in vacuo. Yield: 1 g PBLG-diaminobutane.
¹H-NMR (CDCl₃) (δ relative to solvent peak):
PBLG: 2.2 & 2.6 (β,γ-CH₂), 4.0 (α-CH), 5.0 (benzyl CH₂), 7.3 (phenyl)
BOC signals absent

PBLG-diaminobutane DODASuc (DCC coupling):
170 mg N-succinyl-dioctadecylamine (DODASuc), 90 mg DCC and 10 mg 4-(dimethylamino)pyndinium 4-toluene sulphonate (DPTS) were dissolved in 4 ml dichloromethane. The solution was stirred for 1 hour at roomtemperature. A solution of 0.73 g PBLG-diaminobutane and 40 µl triethylamine in 3 ml chloroform was added. After stirring overnight at room temperature the solution (containing dicyclohexylurea precipitate) was added dropwise to an excess of methanol (ca. 100 ml). The polymeric product was filtered off and dried. Yield: 0.5 g.
¹H-NMR (CDCl₃) (δ relative to solvent peak):
   distearyl signals at 0.8-0.9 (CH₃) and 1.2-1.4 (methylene protons)
   PBLG: 2.2 & 2.6 (β,γ-CH₂), 4.0 (α-CH), 5.0 (benzyl CH₂), 7.3 (phenyl)

### 5.1 PHEG-diaminobutane DODASuc

PHEG-diaminobutane DODASuc was obtained by aminolysis of PBLG-diaminobutane DODASuc with ethanolamine as follows:
0.5 g PBLG-diaminobutane DODASuc and 15 mg 2-hydroxypyridine were dissolved in 4 ml DMF. Then 2 ml ethanolamine was added. After stirring for 24 hours at 40 °C under a nitrogen atmosphere the solution was precipitated into ca. 100 ml diethylether. PHEG-diaminobutane DODASuc was dissolved in water, dialyzed (MWCO 500) and subsequently freeze-dried yielding 0.35 g purified PHEG diaminobutane DODASuc conjugate.
¹H-NMR (DMSO-d6) (δ relative to solvent peak):
   distearyl signals at 0.8-0.85 (C*H*₃) and 1.2-1.5 (methylene protons)
   PHEG: 1.7-2.2 (β,γ-CH₂), 3.1 & 3.3 (hydroxyethyl), 4.2 (α-CH), 4.7 (OH), 7.8 & 8.2 (NH)
From the ratio of the integrals of the distearyl signals and the α-CH signal the PHEG molecular weight was calculated to be ca. 4000.
Maldi-TOF confirms the molecular structure of the PHEG diaminobutane DODASuc conjugate.
Na⁺-adduct: m/z 3064.5 (n=13), 3236.1 (n=14), 3408.7 (n=15), 3580.6 (n=16), 3752.9 (n=17), 3924.7 (n=18), 4096.7 (n=19), 4268.4 (n=20), 4441.1 (n=21), 4613.3 (n=22), 4785.1 (n=23), etc.

### 5.2 Poly-[(2-hydroxypropyl)-L-glutamine] diaminobutane DODASuc

Poly-[(2-hydroxypropyl)-L-glutamine] diaminobutane DODASuc was obtained by aminolysis of PBLG-diaminobutane DODASuc with 2-propanolamine (isopropanolamine) as follows:
0.15 g PBLG-diaminobutane DODASuc and 0.05 g 2-hydroxypyridine were dissolved in 4 ml DMF. Then 1 ml 2-propanolamine was added. After stirring for 24 hours at 40 °C under nitrogen atmosphere the solution was precipitated into ca. 100 ml diethylether. 0.1 g PHisoPG-diaminobutane DODASuc was obtained after drying. Polymer was dissolved in water, dialyzed (MWCO 500) and subsequently freeze-dried yielding purified poly-[(2-hydroxypropyl)-L-glutamine] diaminobutane DODASuc.
¹H-NMR (DMSO-d6) (δ relative to solvent peak):
   distearyl signals at 0.8-0.85 (CH₃) and 1.2-1.5 (methylene protons)
   PHPG: 1.7-2.2 (β,γ-CH₂), 1.0 (CH₃) & 3.0 & 3.3 & 3.7 (hydroxypropyl), 4.2 (α-CH), 4.7 (OH), 7.8 & 8.2 (NH).
   Calculated molecular weight: ca. 4000.

### 5.3 Poly-[(3-hydroxypropyl)-L-glutamine] diaminobutane DODASuc

Poly-[(3-hydroxypropyl)-L-glutamine] diaminobutane DODASuc was obtained by aminolysis of PBLG-diaminobutane DODASuc with 3-propanolamine:
0.3 g PBLG-diaminobutane DODASuc and 0.1 g 2-hydroxypyridine were dissolved in 4 ml DMF. Then 2 ml 3-propanolamine was added. After stirring for 24 hours at 40 °C under nitrogen atmosphere the solution was precipitated into ca. 100 ml diethylether. 0.25 g PHPG5000-diaminobutane DODASuc was obtained after drying. Polymer was dissolved in water, dialyzed (MWCO 500) and subsequently freeze-dried yielding purified poly-[(3-hydroxypropyl)-L-glutamine] diaminobutane DODASuc.
¹H-NMR (DMSO-d6) (δ relative to solvent peak):
   distearyl signals at 0.8-0.85 (C*H*₃) and 1.2-1.5 (methylene protons)
   PHPG: 1.7-2.2 (β,γ-CH₂), 1.5 & 3.1 & 3.3 (hydroxypropyl), 4.2 (α-CH), 4.6 (OH), 7.8 & 8.2 (NH).
   Molecular weight: ca. 5000.
Maldi-TOF:
   Na⁺-adduct: *m*/*z* 3623 (n=15), 3810 (n=16), 3996 (n=17), 4182 (n=18), 4368 (n=19), 4555 (n=20), etc.

### 5.4 Poly-[(4-hydroxybutyl)-L-glutomine] diaminobutane DODASuc

Poly-[(4-hydroxybutyl)-L-glutamine] diaminobutane DODASuc was obtained by aminolysis of PBLG-diaminobutane DODASuc with 4-butanolamine:
0.3 g PBLG-diaminobutane DODASuc and 0.1 g 2-hydroxypyridine were dissolved in 4 ml DMF. Then 2 ml 4-butanolamine was added. After stirring for 48 hours at 40 °C under nitrogen atmosphere the solution was precipitated into ca. 100 ml diethylether. Polymer was dissolved in water, dialyzed (MWCO 500) and subsequently freeze-dried yielding 0.2 g purified poly-[(4-hydroxybutyl)-L-glutamine] diaminobutane DODASuc.
¹H-NMR (DMSO-d6) (δ relative to solvent peak):
   distearyl signals at 0.8-0.85 (CH₃) and 1.2-1.5 (methylene protons)
   PHBG: 1.7-2.2 (β,γ-CH₂), 1.4 & 3.1 & 3.3 (hydroxybutyl), 4.2 (α-CH), 4.5 (OH), 7.8 & 8.2 (NH).
   Molecular weight: ca. 4000

### 5.5 Poly-[(2,3-dihydroxypropyl)-L-glutamine] diaminobutane DODASuc

Poly-[(2,3-dihydroxypropyl)-L-glutamine] diaminobutane DODASuc was obtained by aminolysis of PBLG-diaminobutane DODASuc with 2,3-dihydroxypropylamine:
0.15 g PBLG-diaminobutane DODASuc and 0.06 g 2-hydroxypyridine were dissolved in 3 ml DMF. Then 1 ml 2,3-dihydroxypropylamine was added. After stirring for 1 day at 40 °C under nitrogen atmosphere the solution was precipitated into ca. 100 ml diethylether. Polymer was dissolved in water, dialyzed (MWCO 500) and subsequently freeze-dried yielding 0.1 g purified poly-[(2,3-dihydroxypropyl)-L-glutamine] diaminobutane DODASuc. ¹H-NMR (DMSO-d6) (δ relative to solvent peak):
   distearyl signals at 0.8-0.85 (C*H*₃) and 1.2-1.5 (methylene protons)
   Poly(dihydroxypropyl)G: 1.7-2.2 (β,γ-CH₂), 3.1 & 3.3-3.6 (dihydroxypropyl), 4.2 (α-CH), 4.6 & 4.8 (OH), 7.8 & 8.2 (NH).
   Molecular weight: ca. 4000.

### Example 6

### Cholesteryl-PHEG

To a solution of 0.2 g PBLG-NH₂ and 20 µl triethylamine in 2 ml chloroform was added a solution of 0.07 g cholesteryl chloroformate in 1 ml chloroform. The solution was stirred for ca. one hour at room temperature and then precipitated into diethyl ether. After collecting and drying 0.13 g polymeric product was obtained.

Aminolysis with ethanolamine (2-hydroxypyridine in the role of catalyst) for 1 day at 40 °C yielded cholesteryl-PHEG. The polymeric product was purified by dialysis (MWCO 500).
¹H-NMR (DMSO-d6) (δ relative to solvent peak):
PHEG: 1.7-2.2 (β,γ-CH₂), 3.1 & 3.3 (hydroxyethyl), 4.2 (α-CH), 4.7 (OH), 7.8 & 8.2 (NH) cholesteryl: 0.6-1.6.
Molecular weight: ca. 4000.
Maldi TOF confirms the molecular structure of the cholesteryl-PHEG conjugate
Na⁺-adduct: *m*/*z* 3046 (n=14), 3218 (n=15), 3390 (n=16), 3562 (n=17), 3735 (n=18), 3907 (n=19), 4080 (n=20), etc.

### Example 7

### Poly(2-hydroxyethyl)-DL-glutamine diaminobutane DODASuc

The synthesis is analogous to that of poly(2-hydroxyethyl)-L-glutamine diaminobutane DODASuc (example 5), however differing in a few details:
γ-Benzyl-DL-glutamine NCA was synthesized from a 1:1 mixture of γ-benzyl-L- and γ-benzyl-D-glutamate and crystallized from ethylacetate/hexane (ca. 1:5).
Poly(benzyl-DL-glutamine) diaminobutane BOC was precipitated into water instead of methanol.
Poly(benzyl-DL-glutamine) diaminobutane DODASuc was precipitated into methanol.
NMR spectrum is virtually identical to that of poly(2-hydroxyethyl)-L-glutamine diaminobutane DODASuc (example 5.1).
Molecular weight: ca. 3000.

### Example 8

### PHEG copolymers: poly(HEG-co-glutamic acid) diaminobutane DODASuc; 5% glutamic acid

A solution of 0.14 g PBLG diaminobutane DODASuc, 0.05 g 2-hydroxypyridine, ca. 1 ml ethanolamine in 1.5 ml DMF was stirred under a nitrogen atmosphere for one day at room temperature. The solution was then precipitated into diethylether. The product, partially ethanolamine-aminolyzed PBLG diaminobutane DODASuc (PHEG with 5% benzyl ester side groups), was collected and dried. NMR recorded in DMSO revealed the presence of 5% unreacted benzyl groups.
Said polymer was dissolved in 8.5 ml 1 M NaOH and stirred for 4 hours. The solution was neutralized with 1 N HCl, and then dialyzed (MWCO 500) for a few days. The negatively charged (at physiological pH) copolymer-lipid-conjugate (0.1 g) was obtained after freeze-drying the dialyzed solution.
NMR in DMSO showed full conversion ofbenzylgroups.
MaldiTOF was used to confirm the presence of both glutamic acid and hydroxyethylglutamine repeating units.
Molecular weight: ca. 3500.

### Example 9

### PHEG copolymer: poly(HEG-co-dimethylaminoethylglutamine) diaminobutane DODASuc; 5 % dimethylaminoethyl sidegroups

A solution of 0.25 g PBLG diaminobutane DODASuc, 0.08 g 2-hydroxypyridine, and 1 ml ethanolamine in 2.5 ml DMF was stirred under a nitrogen atmosphere for two days at room temperature. The resulting solution was precipitated into diethylether. The partially ethanolamine-aminolyzed PBLG diaminobutane DODASuc (PHEG with 5 % benzyl ester side groups) was collected and dried. NMR recorded in CDCl₃ revealed the presence of 5 % unreacted benzyl groups.
A solution of 0.16 g of this partially ethanolamine-aminolyzed PBLG diaminobutane DODASuc, 0.06 g 2-hydroxypyridine, 1 ml N,N-dimethylethylenediamine in 2.5 ml DMF was stirred for 1 day under a nitrogen atmosphere at 40 °C. Precipitation into diethylether gave a powder that was collected and dried in vacuo. NMR in DMSO showed full conversion of the remaining benzyl groups. Product was dissolved in water and dialyzed (MWCO 500) for a few days and subsequently freeze-dried. Yield: 0.1 g of positively charged PHEG copolymer-lipid-conjugate.
Molecular weight: ca. 4000.

### Example 10

### Poly(2-hydroxyethyl)-L-asparagine (PHEA) having a stearylamine and heptadecyloctadecylamine end group, respectively

β-benzyl-L-aspartate N-carboxyanhydride (NCA):
A suspension of 5 g β-benzyl L-aspartate in 50 ml distilled THF containing ca. 16 ml of a 20 % phosgene solution in toluene was heated at 60-65 °C (stream of nitrogen gas over the solution). After ca.10 minutes a clear solution was obtained. After ca. 1.5 hours the solution was slowly poured into ca. 140 ml n-hexane. Crystals were formed almost immediately. After further crystallization during one night at -20 °C the NCA crystalline product was isolated. Further crystallizations from THF/hexane and from hot chloroform yielded 4.3 g fine needles (Biopolymers 1976, 15(9) 1869-71).
¹H-NMR (CDCl₃) (δ relative to solvent peak):
   benzyl group: 7.3 (Phenyl), 5.1 (CH₂)
   aspartate NCA: 2.8 & 3.0 (β-CH₂), 4.5 (α-CH), 6.4 (NH)
stearyl-PBLA:
To a solution of 0.95 g β-benzyl L-aspartate NCA in 2 ml DMF was added a solution of 0.04 g stearylamine in 0.5 ml chloroform. After stirring for several hours at 60°C the cloudy solution was precipitated into methanol. After drying 0.56 g poly(benzyl L-aspartate) stearylamine was obtained.
heptadecyl octadecyl-PBLA:
To a solution of 0.5 g β-benzyl L-aspartate NCA in 2 ml DMF was added 0.1 g 1-heptadecyl octadecylamine in ca. 1 ml chloroform. After stirring for 3 days at room temperature the cloudy solution was precipitated into methanol. Yield: 0.2 g polymeric product PBLA-heptadecyl octadecyl amine.
stearyl-/heptadecyl octadecyl-PHEA:
Aminolysis of the above PBLA-conjugates using ethanolamine and 2-hydroxypyridine as a catalyst at 40 °C for 1 day, followed by precipitation into diethyl ether, yields the water-soluble poly(hydroxyethyl) L-asparagine (PHEA), containing a stearyl and a heptadecyl octadecyl tail respectively. The lipid-polymer-conjugates were purified by dialysis (MWCO 500).
stearyl-PHEA:
Maldi TOF: Na⁺ adduct *m*/*z* 2823 (n=16), 2981 (n=17), 3139 (n=18), 3297 (n=19), 3455 (n=20), 3613 (n=21), etc.

From Maldi TOF it was concluded that each PHEA chain contains a free amino end group.
heptadecyl octadecyl-PHEA:
NMR (DMSO-d6) (δ relative to solvent peak):
   heptadecyl octadecyl: 0.8 & 1.2
PHEA: 2.2-2.6 (β-CH₂), 3.1 & 3.4 (hydroxyethyl), 4.5 (OH + α-CH), 7.8 & 8.3 (NH)
Molecular weights: ca. 6000.

### Example 11

### Poly(2-hydroxyethyl)-L-asparagine DODASuc

PBLA DODASuc:
To a solution of 1.7 g β-benzyl L-aspartate N-carboxyanhydride (NCA) in 5 ml DMF was added 0.2 ml of a 2 M solution of methylamine in THF. The clear solution was stirred for one day and then precipitated into a mixture of methanol (ca. 100 ml) and water (250 ml). Yield 1.3 g PBLA, containing a methyl amide and an amino end group.
A solution of 0.4 g PBLA, 30 mg DCC, 10 mg DPTS and 100 mg N-succinyl-distearylamine in 5 ml DMSO and 1 ml chloroform was stirred for one day and then precipitated into water. Polymeric product was stirred/washed with diethyl ether and dried.

PHEA DODASuc
Aminolysis of PBLA DODASuc with ethanolamine (using 2-hydroxypyridine as a catalyst) in DMF solution at 40 °C for 1 day yielded PHEA DODASuc (0.2 g after dialysis and freeze-drying).
¹H-NMR (DMSO-d6) (δ relative to solvent peak):
distearyl: 0.8 (CH₃), 1.2 (CH₂), 1.4 (CH₂-N)
PHEA: 2.4-2.8 (β-CH₂), 3.2 & 3.4 (hydroxyethyl), 4.6 (α-CH + OH), 7.8-8.5 (NH)
Calculated molecular weight: ca. 3000.
Maldi TOF confirms the molecular structure of the PHEA DODASuc conjugate:
Na⁺-adduct: *m*/*z* 2084 (n=9), 2243 (n=10), 2401 (n=11), 2559 (n=12), 2718 (n=13), 2876 (n=14), etc.

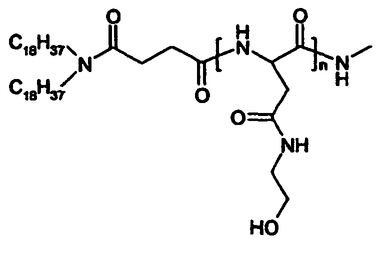

### Example 12

### Poly(2-hydroxyethyl)-L-asparagine DSPESuc conjugate

Amino-terminated PHEA was obtained after aminolysis of PBLA (polybenzyl-L-aspartate), obtained from the methylamine-initiated polymerization of benzyl-L-aspartate NCA. Succinylated DSPE (synthesis analogous to the one described for DPPE in JACS, 116, 8485 (1994)) was first converted to its NHS ester in-situ using DCC (dicyclohexylcarbodiimide):
A solution of 70 mg succinylated DSPE, 20 mg NHS (N-hydroxysuccinimide), 5 mg DMAP and 30 mg DCC (dicyclohexylcarbodiimide) in 2 ml dichloromethane was stirred for ca. 3-4 hours. To this mixture was added a solution of 0.13 g of the amino-terminated PHEA (molecular weight ca. 4000) in 2 ml DMSO. After stirring overnight the mixture was precipitated into ether. The precipitate was collected and dissolved in water and dialyzed (MWCO 500) for a few days. After freeze-drying ca. 80 mg PHEA-DSPE was obtained.
¹H-NMR (DMSO-d6) (δ relative to solvent peak):
   DSPE: 0.8 (CH₃), 1.2 (CH₂), 1.4 (CH₂-N)
   PHEA: 2.4-2.8 (β-CH₂), 3.2 & 3.4 (hydroxyethyl), 4.6 (α-CH + OH), 7.8-8.4 (NH)
   Calculated molecular weight: ca. 4000

### Example 13

### Poly(DL-serine) DODASuc

O-benzyl-DL-serine N-carboxyanhydride (NCA):
A suspension of 2.5 g O-benzyl-DL-serine in 30 ml distilled (dry) THF containing ca. 10 ml of a 20% phosgene solution in toluene was heated at 60-65 °C (stream of nitrogen gas over the solution). After ca. 5 minutes a clear solution was obtained. After ca. 1.5 hours the solution was slowly poured into ca. 100 ml n-hexane. The product separated as an oil. The solvent was decanted and the oil was dissolved in ca. 25 ml ethylacetate to which 100 ml hexane was slowly added. After violently shaking the flask and refrigerating at -20 °C O-benzyl-DL-serine NCA started to crystallize. Similar recrystallizations from ethylacetate/hexane and/or from chloroform/hexane yielded 2 g crystalline material (Biopolymers 1976,15(9) 1869-71).
¹H-NMR (CDCl₃) (δ relative to solvent peak):
   benzyl group: 4.5 (CH₂), 7.2 (Phenyl)
   serine NCA: 3.7 (β-CH₂), 4.4 (α-CH), 5.8 (NH)
poly(O-benzyl-Dlrserine):
To a solution of 0.9 g 0-benzyl-Dlserine NCA in 2.5 ml DMF was added 0.08 ml of a solution of 2 M methylamine in THF. After stirring for several hours at room temperature the solution became cloudy and viscous. After 1 day the viscous, "crystallized" solution was mixed with methanol/water to precipitate the polymeric product completely. Yield: 0.6 g polymeric product poly(O-benzyl-DL-serine).
¹H-NMR in DMSO-d6 (δ relative to solvent peak):
benzyl groups: 4.4 (CH₂), 7.2 (Phenyl)
polyserine: 3.5 (β-CH₂), 4.7 (α-CH), 8.2 (NH)
Poly(O-benzyl-DL-serine) DODASuc:
150 mg N-succinyl-dioctadecylamine (DODASuc), 80 mg DCC and 5 mg DPTS were dissolved in 4 ml chloroform. The solution was stirred for 1 hour at room temperature. A solution of 0.6 g poly(O-benzyl-DL-serine) and ca. 50 µl triethylamine in ca. 5 ml chloroform was added. After stirring overnight at room temperature the solution (containing dicyclohexylurea precipitate) was added dropwise to an excess of methanol (ca. 100 ml). The polymeric product was filtered off and dried. Yield: 0.4 g.
¹H-NMR in DMSO-d6 (δ relative to solvent peak):
distearyl: 0.8 (CH₃), 1.2 (CH₂), 1.6 (CH₂-N)
benzyl groups: 4.4 (CH₂), 7.2 (Phenyl)
polyserine: 3.5 (β-CH₂), 4.7 (α-CH), 8.2 (NH)
Poly(DL-serine) DODASuc:
0.1 g poly(O-benzyl-DL-serine) DODASuc was dissolved in ca. 4 ml of a 33% HBr/AcOH solution and stirred for 1 hour. The solution was then precipitated into water. The polymeric precipitate was filtered off, washed with water, collected and subsequently dissolved (1-2 hours) in 4 ml 1M NaOH. The resulting solution was neutralized with 1 N HCl solution, and then dialyzed (MWCO 500) for several days. The dialyzed solution was freeze dried. Yield: 20 mg poly(DL-serine) DODASuc..
¹H-NMR in DMSO-d6 (δ relative to solvent peak):
distearyl: 0.8 (CH₃),1.2 (CH₂), 1.6 (CH₂-N)
polyserine: 3.6 (β-CH₂), 4.3(OH), 5.0 (α-CH), 8.0 (NH)

From the ratio of integrals of the distearyl signals and the α-CH signal the polyserine molecular weight was calculated to be ca. 1500.

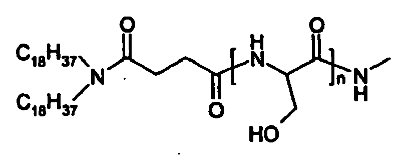

### Example 14

### Poly-L-threonine DODASuc

The synthesis is analogous to the synthesis of poly(D,L-serine) DODASuc and was done via O-benzyl-L-threonine N-carboxyanhydride (NCA), starting from O-benzyl-L. thraonine.HCl and phosgene.
Poly-L-threonine DODASuc][M = ca. 2000):
¹H-NMR in DMSO-d6 (δ relative to solvent peak):
   distearyl: 0.8 (CH₃), 1.2 (CH₂), 1.6 (CH₂-N)
   polythreonine: 1.0 (CH₃), 4.0 (β-CH), 4.3(α-CH), 5.0(OH), 7.8 (NH)

### Example 15

### Poly(D,L-methionine sulfoxide) DODASuc

DL-methionine N-carboxyanhydride (NCA):
A suspension of 2.5 g DL-methionine in 40 ml distilled (dry) THF containing ca. 15 ml of a 20 % phosgene solution in toluene was heated at 60-65 °C (stream of nitrogen gas over the solution). Almost immediately a clear solution was formed. After ca. 1 hour the solution was slowly poured into ca.140 ml n-hexane. DL-methionine NCA was crystallized at -20 °C (takes a few days). Recrystallization from ethylacetate/hexane yielded ca. 0.7 g crystalline material (Biopolymers 1976, 15(9) 1869-71)
   ¹H-NMR (CDCl₃) (δ relative to solvent peak):
   methionine NCA: 2.0-2.4 (β-CH₂ + CH₃), 4.5(α-CH), 6.8 (NH)
poly(DL-methionine):
To a solution of 0.7 g DL-methionine NCA in 2.5 ml DMF was added 0.1 ml of a solution of 2 M methylamine in THF. After 1 day the cloudy solution was precipitated into ca. 100 ml methanol and subsequently dried. Yield: 0.33 g polymeric product poly (DL-methionine). ¹H-NMR in CDCl₃/TF-d (δ relative to solvent peak):
   polymethionine: 2.0-2.3 (CH₂ & CH₃), 2.6 (CH₂), 4.7 (α-CH).
Poly(DL-methionine) DODASuc:
80 mg N-succinyl-dioctadecylamine (DODASuc), 45 mg DCC and 5 mg DPTS were dissolved in 2 ml chloroform. The solution was stirred for 1 hour at room temperature. A solution of 0.33 g poly(DL-methionine) and ca. 20 µl triethylamine in ca. 2.5 ml DMSO was added. After stirring overnight at room temperature the solution (containing dicyclohexylurea precipitate) was added dropwise to an excess of methanol (ca. 100 ml). The polymeric product was filtered off and dried. Yield: 0.22 g.
   ¹H-NMR in DMSO-d6 (δ relative to solvent peak):
   distearyl: 0.8 (CH₃), 1.2 (CH₂), 1.4 (CH₂-N)
   polymethionine: 1.8 (β-CH₂), 2.0 (CH₃), 2.4 (γ-CH₂), 4.4 (α-CH), 8.1 (NH)
Poly(DL-methionine sulfoxide) DODASuc:
Mono-oxidation of poly(DL-methionine) DODASuc:
   A solution of 0.3 g sodium periodate in 2 ml water was slowly added to a suspension of 0.22 g poly(DL-methionine) diaminobutane DODASuc in ca. 6 ml acetic acid. The resulting orange/red solution (which was formed after a few hours) was stirred for 1 night. Then ca. 15 ml water was added and the resulting orange/red solution was dialyzed (MWCO 500) for several days. After freeze-drying 0.25 g product was obtained.
   ¹H-NMR in D₂O (δ relative to solvent peak):
   distearyl: 0.7 (CH₃), 1.2 (CH₂) (broad peaks)
   polymethionine sulfoxide: 2.0 (β-CH₂), 2.5 (CH₃), 2.8 (γ-CH₂), 4.3 (α-CH), 8.5 (NH)
   Calculated molecular weight: ca. 4000.

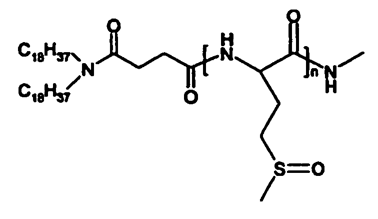

### Example 16

### Poly(DL-glutamine) DODASuc

A poly(DL-glutamine) DODASuc conjugate was synthesized by Ansynth Service B.V. using a solid phase peptide synthesis method (ca. 50 mg scale). Poly(DL-glutamine) (n=20) bound to a resin was built up step by step using Fmoc-protected aminoacids. To the N-terminus was coupled N-succinyl-distearylamine. The C-terminus was transformed to an amide. ¹H-NMR spectrum confirmed the structure.
¹H-NMR in DMSO-d6 (δ relative to solvent peak):
distearyl: 0.8 (CH₃), 1.2 (CH₂), 1.4 (CH₂-N)
polyglutamine: 1.7 - 2.2 (β,γ-CH₂), 4.2 (CH), 6.8 & 7.3 (NH₂), 8.2 (NH)

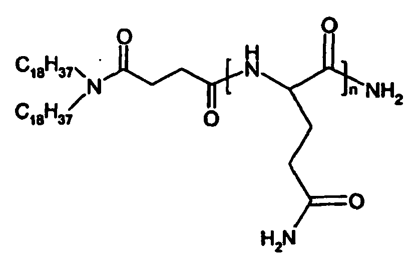

### Example 17

### Poly(DL-asparagine) DODASuc

A poly(DL-asparagine) DODASuc conjugate was synthesized by Ansynth Service B.V. using a solid phase peptide synthesis method starting from Fmoc-protected aminoacids (ca. 50 mg scale). Poly(DL-asparagine) (n=20) bound to a resin was built up step by step. To the N-terminus was coupled N-succinyl-disteatylamine. The C-tenninus was transformed to an amide. ¹H-NMR spectrum recorded in DMSO confirmed the structure.
¹H-NMR in DMSO-d6 (δ relative to solvent peak):
distearyl: 0.8 (CH₃), 1.2 (CH₂), 1.4 (CH₂-N)
polyasparagine: 2.5 (CH₂), 4.5 (CH), 7.0 & 7.4 (NH₂), 8.1 (NH)

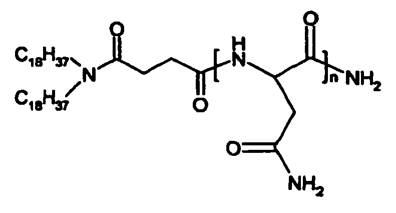

### Example 18

### Poly-(D,L-alanine) DODASuc

95 mg poly-DL-alanine (Sigma; MW: ca. 2000) was dissolved in 4 ml DMSO. 40 µl triethylamine was added to this solution. Subsequently, this solution was added to a solution of 0.1 g DODASuc, 70 mg DCC and 5 mg DPTS in 2 ml chloroform that has been stirred for 1 hour. After stirring for 1 day the mixture was precipitated into a methanol/diethylether mixture. The precipitate was filtered off and dried.
¹H-NMR recorded in DMSO:
Distearyl: 0.8 *(CH₃),* 1.2 (C*H₂),*1.4 (C*H₂*N)
Polyalanine: 1.2(*CH₃*), 4.2 (C*H*), 8.0 (N*H*).

### Example 19

### Copolypeptide of β-alanine and hydroxyethyl L-glutamine

DODASuc-(β-Ala)₃-Glu(OBzl)-(β-Ala)₄-Glu(OBzl)-(β-Ala)₃-Glu(OBzl)-(β-Ala)₂-NH₂: Copolypeptide of β-alanine and benzyl L-glutamate was synthesized via a solid phase method by Ansynth Service B.V. starting from Fmoc-protected monomers
C-terminus: amide; N-terminus: DODASuc.
DODASuc-(β-Ala)₃-HEG(β-Ala)₄-HEG-(β-Ala)₃-HEG-(β-Ala)₂-NH₂:
Afterwards the benzyl glutamate units were converted to hydroxyethyl glutamine (HEG) ones by an aminolysis (ethanolamine) reaction carried out in DMF.
¹H-NMR recorded in DMSO revealed absence/conversion of benzyl groups.

### Example 20

### Preparation of PHEG-stearylsmine-containing liposomes

33.8 mg of egg phosphatidylcholine (EPC) (Lipoid Ludwigshafen), 9.67 mg of cholesterol (Sigma Aldrich) and 30.0 mg of poly-[N-(2-hydroxyethyl)-L-glutamine]-stearylamine (PHEG-stearylamine) (synthesised) were weighed and transferred in a 50 ml round-bottom flask. 500 kBq of tritium-labeled cholesteryloleylether was added as a lipid marker. The lipids and the label were dissolved in about 10 ml of ethanol. Thereafter evaporating to dryness in a Rotavapor during 1 hour under vacuum at 40°C, followed by flushing with nitrogen gas during 1 hour took place.

PBS was added to the dry lipid film and shaken during one hour in the presence of glass beads in order to enable complete hydration of the lipid film.

The liposomal suspension was transferred to an extruder (Avestin, maximum volume 15 ml) and extruded under pressure, using nitrogen gas, 6 times through 2 polycarbonate filters one placed on top of the other, having a pore size of 200 and 100 nm respectively, and 18 times through filters having a pore size of 100 nm and 50 nm respectively. Subsequently the liposomal suspension was dialysed in a dialysing compartment (Slide-A-Lyzer, 10,000 MWCO) 2 times during 24 hours against 1 liter of sterilised PBS.

The mean particle size of the liposomes was determined by means of light scattering (Malvern Zeta-sizer) and was found to be 93.6 ± 0.9 nm, the polydispersity index being 0.099 ± 0.02. The lipid loss during preparation of the liposomes was 25 %, determined by comparing the final radioactivity of the preparation with the activity before the extrusion procedure. The suspension of liposomes was stored in a nitrogen atmosphere at 4°C.

### Example 21

### Preparation of further lipid-polymer-conjugates-containing liposomes

Liposomes were prepared using the film method, as described in example 20. Instead of egg phosphatidylcholine dipalmitoyl phosphatidylcholine was used. 5 mM HEPES buffer was added to the dry lipid film and shaken during 5 minutes in the presence of glass beads in order to enable complete hydration of the lipid film. The liposomes were sized by extrusion 12 times through 2 stacked PC membranes having pore sizes of 100 and 200 nm. The resulting liposome dispersions were dialysed (MWCO 10,000) and average particle sizes were determined using dynamic light scattering technique. See table 1 for the properties of the liposomal preparations.

### Example 22

### Comparative kinetics of ³H-labelled Lipid-polymer-conjugates incorporated into liposomes after a single intravenous injection to the rat

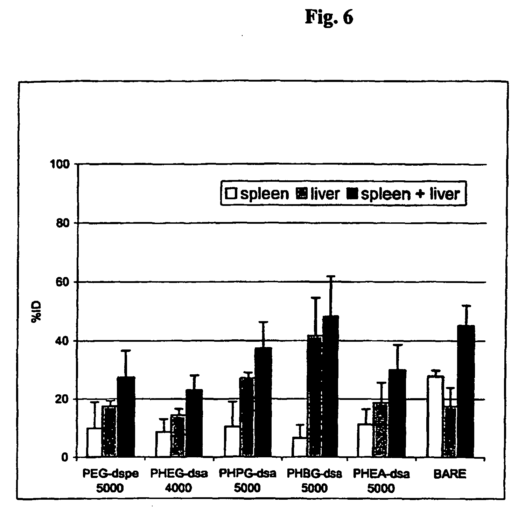

Male rats (Wistar, Crl: (WI) BR (outbred, SPF-Quality) (Charles River, Sulzfeld, Germany)) had free access to standard pelleted laboratory animal diet (Altromin, code VRF 1, Lage, Germany) and to tap-water. Single-dose intravenous injection of liposomal preparations, each containing ³H-labelled cholesteryloleylether (Amersham) having 40-50 kBq of radioactivity, (compositions per 50 µmol lipid are shown in Table 1) was given into the tailvein. Total Lipid administered was 5 µmol, except in the cases indicated.
Blood samples were collected from the tail vein of each rat at the following time points post-dose: 5 minutes and 4, 24 and 48 hours. The amount of sample collected was approx. 300 µl per sampling event.
Sampled blood was transferred into heparinised tubes and stored at -20 °C.
A single aliquot of 100 µl was solubilised according to the following method:
- 100 µl was transferred to a scintillation vial (20 ml).
- 100 µl of Solvable was added. This mixture was incubated for at least 1 hour.
- 100 µl of 1 mM EDTA and 200 µl H₂O₂ 30% were added. This mixture was incubated for 24 hours at room temperature and overnight at 50 °C thereafter.
- Ultima Gold (10 ml) was added as the scintillation fluid.
- Radioactivity was measured by LSC.
All radioactivity measurements were performed using a Packard scintillation counter (1900TR). Counting time was to a statistical precision of ± 0.2% or a maximum of 5 minutes, whichever comes first. The Packard 1900TR was programmed to automatically subtract background and convert counts per minute (CPM) to disintegrations per minute (DPM).
For some of the preparations mentioned the liver and spleen of the rats were dissected 48 hours after injection and liposomes localisation was assessed according to the following method:
The organs were homogenised and the homogenates diluted to 25 ml (liver) or 5 ml (spleen). 1 ml of the diluted homogenates was transferred to scintillation vials to which subsequently were added:
   - 200 ml Solvable (mixed and sample incubated at 50 °C overnight)
   - 200 ml 0.5 M EDTA solution
   - 250 ml of H₂O₂ (30%) solution (incubated at 50 °C overnight)
   - 10 ml Ultima Gold scintillation liquid (vortexed and sample incubated for 24 hours).
Thereafter the samples were counted in a beta-scintillation counter for 10 minutes. Results for some liposomal preparations are shown in Figure 6.
The compositions of the liposomal preparations, prepared according to Example 21, and the results, obtained in the in vivo test of this example, are shown in Table 1. The increase of blood circulation time was assessed, wherein:
   - Good means effect on circulation time comparable to that shown by PEG-DSPE-containing liposomes.
   - Moderate means effect on circulation time in between those shown by PEG-DSPE-containing liposomes and bare liposomes without polymer coating.
   - Slightly means effect on circulation time under the current conditions almost similar to that shown by bare liposomes.

**Table 1**

| Composition of liposomes per 50 µmol lipid and properties | | | | | |
|---|---|---|---|---|---|
| DPPC (mg) | Cholesterol (mg) | Lipid-Polymer-conjugate of (mg) | Average particle size (nm) | Polydispersity index | Increase of blood circulation time |
| 23.1 | 6.4 | Ex. 3:10 | 153 ± 0.5 | 0.056 | Good |
| 23.1 | 6.4 | Ex. 2: 30 | 143 ± 2 | 0.205 | Moderate |
| 23.1 | 6.4 | Ex. 5.1:11.2 | 140.3 ± 2.2 | 0.090 | Good |
| 23.1 | 6.4 | Ex. 5.2: 13.8 | 153.0 ± 0.9 | 0.090 | Moderate |
| 23.1 | 6.4 | Ex. 5.4:11.2 | 148.2 ± 1.7 | 0.071 | Slightly |
| 23.1 | 5.5 | Ex. 6: 11.0 | 138.7 ± 1.8 | 0.116 | Slightly |
| 23.1 | 6.4 | Ex. 11: 8.9 | 146.0 ± 2.1 | 0.092 | Good |
| 23.1 | 6.4 | Ex. 11:13.8 | 147.0 ± 1.1 | 0.068 | Good |
| 23.1 | 6.4 | Ex. 11: 8.9 | 141.7 ± 2.3 | 0.044 | Good |
| 23.1 | 6.4 | Ex. 5.2: 11.2 | 163.9 ± 3.0 | 0.068 | Moderate |
| 23.1 | 6.4 | Ex. 20: 2.2 | 171.6 ± 2.5 | 0.167 | Slightly |
| 23.1 | 6.4 | Ex. 8: 10.1 | 180.7 ± 6.1 | 0.113 | Slightly |
| 23.1 | 6.4 | Ex. 5.1:11.2 | 159.0 ± 3.8 | 0.073 | Good § |
| 23.1 | 6.4 | Ex. 11: 8.9 | 152.9 ± 3.4 | 0.050 | Good § |
| 23.1 | 6.4 | Ex. 9: 11.2 | 170.3 ± 2.5 | 0.039 | Moderate |
| 23.1 | 6.4 | Ex. 9: 2.24 + Ex. 5.1: 8.96 | 166.0 ± 0.9 | 0.056 | Moderate |
| 23.1 | 6.4 | Ex. 8: 10.1 | 167.7 ± 0.6 | 0.170 | Slightly |
| 23.1 | 6.4 | Ex. 15: 11.2 | 159.9 ± 2.3 | 0.062 | Good |
| 23.1 | 6.4 | Ex. 13: 5.0 | 162.7 ± 1.6 | 0.159 | Slightly |
| 23.1 | 6.4 | Ex. 16: 8.8 | 156.3 ± 3.2 | 0.059 | Moderate |
| 25.0 | 6.4 | Ex. 17: 8.8 | 164.7 ± 4.0 | 0.116 | Moderate |
| 23.1 | 6.4 | Ex. 7: 8.8 | 159.0 ± 3.8 | 0.073 | Slightly |
| 23.1 | 6.4 | Ex. 19: 8.8 | 152.9 ± 3.4 | 0.050 | Moderate |
| 23.1* | 6.4* | Ex. 11: 8.8* | 167.7 ± 0.6 | 0.170 | Good |
| 23.1** | 6.4** | Ex. 11:8.8** | 149.9 ± 2.3 | 0.062 | Good |
| 23.1 *** | 6.4*** | Ex. 11:8.8*** | 162.7 ± .024 | 0.159 | Slightly |
| 23.1 | 6.4 | PEG-DSPE:6.9 | 156.3±3.2 | 0.059 | Good |
| 23.1* | 6.4* | PEG-DSPE: 8.8* | 170.3±2.5 | 0.039 | Good |
| 23.1** | 6.4** | PEG-DSPE: 6.9** | 166.0±0.9 | 0.056 | Good |
| 23.1*** | 6.4*** | PEG-DSPE: 6.9*** | 170.5±0.3 | 0.110 | <Slightly |

| | | | | | |
|---|---|---|---|---|---|
| * : Total Lipid administered 0.5 µmol (see Figures 1 and 2) ** : Total Lipid administered 0.05 µmol (see Figures 1 and 2) ***: Total Lipid administered 0.005 µmol (see Figures 1 and 2) §: when a second injection (TL 1 µmol) was given after 1 week, a reduction was seen in the circulation time for liposomes, containing the lipid-polymer-conjugate of example 5.1. The liposomes, containing the conjugate of example 11, also showed such reduction, but for 2 animals this effect was observed to be moderate. | | | | | |

### Example 23

### Preparation of liposomes containing a lipid-polymer-conjugate and prednisolone phosphate

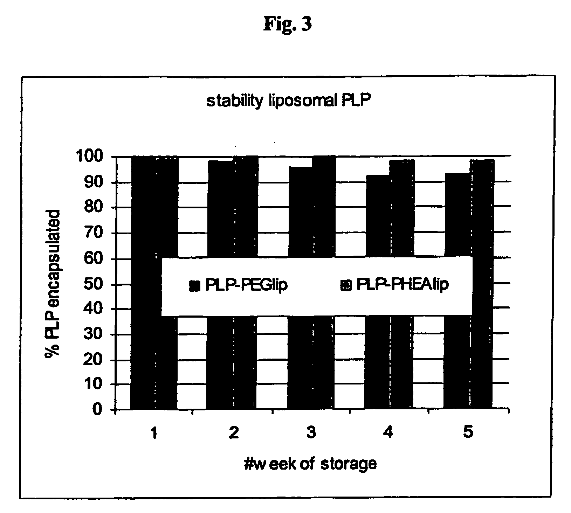

750 mg of dipalmitoyl phosphatidylcholine (DPPC) (Lipoid Ludwigshafen), 220.0 mg of cholesterol (Sigma Aldrich) and 270.0 mg of the lipid-polymer conjugate of example 11 and 750 mg of dipalmitoyl phosphatidylcholine, 250.8 mg of cholesterol and 267.6 mg of PEG-distearoylphosphatidylethanol-amine (PEG-DSPE) (Avanti Polar Lipids) respectively were weighed and mixed in a 100 ml round-bottom flask. The lipids were dissolved in about 30 ml of a 1:1 mixture of methanol and chloroform (lipid-polymer-conjugate of example 14) or ethanol (PEG-DSPE). Thereafter evaporating to dryness in a Rotavapor during 1 hour under vacuum at 40°C, followed by flushing with nitrogen gas during 1 hour took place.
1200 mg of prednisolon disodium phosphate (PLP) (OPG Nieuwegein) were weighed and dissolved in 12 ml of sterilised PBS. The solution was added to the dried lipid films and shaken during one hour in the presence of glass beads in order to enable complete hydration of the lipid films.
The liposomal suspensions were transferred to an extruder (Avestin, maximum volume 15 ml) and extruded under pressure, using nitrogen gas, 6 times through 2 pore filters one placed on top of the other, having a pore size of 200 and 100 nm respectively, 100 and 50 nm respectively and 50 and 50 nm respectively. Subsequently the liposomal suspensions were dialysed in a dialysing compartment (Slide-A-Lyzer, 10.000 MWCO) 2 times during 24 hours against 1 liter of sterilised PBS.
The mean particle size of the liposomes was determined by means of light scattering (Malvern Zeta-sizer) and was found to be about 85 and 90 nm respectively, the polydispersity index being < 0.1. The encapsulation efficiency of the prednisolone phosphate was determined by means of a HPLC method and was found to be 2.6%. The suspensions of liposomes were stored in a nitrogen atmosphere at 4°C and found to be stable for at least 5 weeks, wherein the lipsomomal preparations, containing the lipid-polymer-conjugate of example 14 performed slightly better than the liposomal preparations, containing the reference lipid-polymer-conjugate PEG-DSPE (see figure 3).

### Example 24

### Assessment of circulation time and therapeutic efficacy in rat adjuvant-induced arthritis model of prednisolone phosphate-containing liposomal formulations

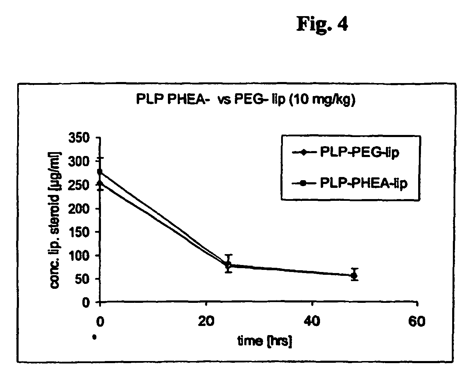

Lewis rats were immunised subcutaneously at the tail base with heat-inactivated *Mycobacterium tuberculosis* in incomplete Freund's adjuvant Paw inflammation started between 9 and 12 days after immunization, reached maximum severity approximately after 20 days, and then gradually resolved.
Assessment of the disease was performed by visually scoring paw inflammation severity from day 10 until day 35 after immunisation. When paw inflammation scores were about to reach values halfway the maximal score (day 14-15), all rats were divided in groups of 5 with equal average scores and treated with a single intravenous injection of:
1. 10 mg/kg PLP in PHEA- DODASuc liposomes, as prepared according to example 23 or
2. 10 mg/kg PLP in PEG-DSPE liposomes, as prepared according to example 23 (reference) or
3. PBS (control).
At t=0, 24 and 48 hours blood samples were collected and assayed for the plasma concentration of liposomal PLP.
The circulation behavior of both PHEA- and PEG-liposomes in blood is shown by the plasma concentration profiles of PLP, which are depicted in the figure 4 Both liposome types perform equally well concerning circulation half life.

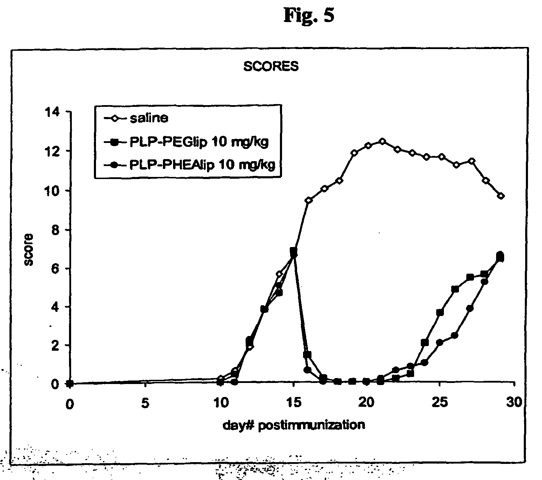

Figure 5 shows the therapeutic activity in rat adjuvant arthritis of 10 mg/kg PLP-PHEA-and 10 mg/kg PLP-PEG-liposomes versus saline-treated rats as controls.

## Claims

1. A lipid-polymer conjugate, obtainable from an amphiphilic lipid, consisting of at least one hydrophobic apolar moiety and a hydrophilic polar head group, and a polymer or a monomeric precursor therefor, having an N- and a C-terminal end group, **characterised in that** the polymer is a poly-(amino acid), a poly-(amino acid derivative) or a poly-(amino acid analogue) and that the lipid is attached to the N or C terminal end group of the polymer, the polymer having the following formula:
-[NHCHR(CH₂)ₘCO]ₙ-
wherein:
-R is defined as:
-H, -CH₃ , -CHCH₃OR₁, -(CH₂)ₓOR₁, -(CH₂)ₓ-CO-NHR₁, -(CH₂)ₓ-NH-CO-R₁, -(CH₂)ₓ-SO_{y}CH₃, or -(CH₂)ₓCOOH;
-R₁ is H or (C₁-C₄)alkyl, substituted with one or more hydroxy groups or one di(C₁-C₄)alkylamine group;
x is 0-4;
m = 1 or 0 and
y = 1 or 2.

2. Conjugate according to claim 1, **characterised in that** the amphiphilic lipid is selected from the group consisting of phospholipids, glycolipids, ceramides, cholesterol and derivatives, saturated or unsaturated, branched or straight chain C₈-C₅₀ mono- or di-alkylamines, arylalkylamines, cycloalkyl alkylamines, alkanols, aldehydes, carbohalides or alkanoic acids and the anhydrides thereof and **in that** the total number of C-atom is 25 or higher.

3. Conjugate according to claim 2, **characterised in that** the amphiphilic lipid contains at least two hydrophobic apolar moieties.

4. Conjugate according to claim 3, in that the amphiphilic lipid is selected from the group consisting of 1-heptadecyloctadecylamine, N-succinyl-diocta-decylamine and distearylphosphatidylethanolamine.

5. Conjugate according to any of the preceding claims, **characterised in that** the polymer has a χ-parameter of ≤ 0.65, preferably ≤ 0.5 in water.

6. Conjugate according to any of the preceding claims, **characterised in that** the polymer consists of α-amino acids and derivatives or analogues thereof

7. Conjugate according to any of the preceding claims, **characterised in that** the polymer contains no substantial amount of charged groups within a physiological pH of 4-8..

8. Conjugate according to claim 7, **characterised in that** the polymer consists of amino acid monomers, amino acid analogue monomers or amino acid derivative monomers, that are neutral or that have been neutralised at a physiological pH of 4-8.

9. Conjugate according to any one of the preceding claim **characterised in that** the polymer has a molecular weight between 500 and 75,000, preferably between 2,000 and 15,000.

10. Conjugate according to any one of the preceding claims, **characterised in that** the polymer is biodegradable.

11. Conjugate according to claim 1, **characterised in that** the polymer is poly[N-(2-hydroxyethyl)]-L-glutamine.

12. Conjugate according to claim 1, **characterised in that** the polymer is poly(2-hydroxyethyl)-L-asparagine.

13. Conjugate according to claim 1, **characterised in that** the polymer is poly(D,L-methionine sulfoxide).

## Patentansprüche

1. Lipid-Polymerkonjugat, erhältlich aus einem amphiphilen Lipid, bestehend aus mindestens einer hydrophoben apolaren Gruppierung und einer hydrophilen polaren Kopfgruppe und einem Polymer oder einem monomeren Vorläufer dafür mit einer N- und einer C-terminalen Endgruppe, **dadurch gekennzeichnet, dass** das Polymer eine Poly(aminosäure), ein Poly(aminosäurederivat) oder ein Poly(aminosäureanaloges) ist und dass das Lipid an die N- oder C-terminale Endgruppe des Polymers gebunden ist, wobei das Polymer die folgende Formel aufweist:
-[NHCHR(CH₂)ₘCO]ₙ-
worin:
-R definiert ist als:
- H, -CH₃, -CHCH₃OR₁, -(CH₂)ₓOR₁, -(CH₂)ₓ-CO-NHR₁, (CH₂)ₓ-NH-CO-R₁, -(CH₂)ₓ-SO_{y}CH₃, oder -(CH₂)ₓCOOH;
-R₁ H oder (C₁-C₄) alkyl, substituiert mit einer oder mehreren Hydroxygruppen oder einer Di(C₁-C₄)alkylaminogruppe;
x 0-4 ist;
m = 1 oder 0 und
y = 1 oder 2.

2. Konjugat nach Anspruch 1, **dadurch gekennzeichnet, dass** das amphiphile Lipid ausgewählt ist aus der Gruppe bestehend aus Phospholipiden, Glycolipiden , Ceramiden, Cholesterin und Derivaten, gesättigt oder ungesättigt, verzweigtkettigen oder geradkettigen C₈-C₅₀-Mono- oder Dialkylaminen, Arylalkylaminen, Cycloalkylalkylaminen, Alkanolen, Aldehyden, Carbohalogeniden oder Alkansäuren und den Anhydriden davon und **dadurch gekennzeichnet, dass** die Gesamtzahl an C-Atomen 25 oder höher ist.

3. Konjugat nach Anspruch 2, **dadurch gekennzeichnet, dass** das amphiphile Lipid mindestens zwei hydrophobe apolare Gruppierungen enthält.

4. Konjugat nach Anspruch 3, **dadurch gekennzeichnet, dass** das amphiphile Lipid ausgewählt ist aus der Gruppe, bestehend aus 1-Heptadecyloctadecylamin, N-Succinyl-diocta-decylamin und Distearylphosphatidylethanolamin.

5. Konjugat nach einem der vorhergehenden Ansprüche, **dadurch gekennzeichnet , dass** das Polymer einen χ-Parameter von ≤ 0,65, vorzugsweise von ≤ 0,5 in Wasser aufweist.

6. Konjugat nach einem der vorhergehenden Ansprüche, **dadurch gekennzeichnet, dass** das Polymer aus α-Aminosäuren und Derivaten oder Analogen davon besteht.

7. Konjugat nach einem der vorhergehenden Ansprüche, **dadurch gekennzeichnet, dass** das Polymer keine wesentliche Menge an geladenen Gruppen innerhalb eines physiologischen pH-Werts von 4-8 enthält.

8. Konjugat nach Anspruch 7, **dadurch gekennzeichnet, dass** das Polymer aus Aminosäuremonomeren, Monomeren von Aminosäureanalogen oder Monomeren von Aminosäurederivaten besteht, die neutral sind oder auf einen physiologischen pH von 4-8 neutralisiert worden sind.

9. Konjugat nach einem der vorhergehenden Ansprüche, **dadurch gekennzeichnet, dass** das Polymer ein Molekulargewicht zwischen 500 und 75.000, vorzugsweise zwischen 2.000 und 15.000 aufweist.

10. Konjugat nach einem der vorhergehenden Ansprüche, **dadurch gekennzeichnet, dass** das Polymer bioabbaubar ist.

11. Konjugat nach Anspruch 1, **dadurch gekennzeichnet, dass** das Polymer Poly[N-(2-hydroxyethyl)]-L-glutamin ist.

12. Konjugat nach Anspruch 1, **dadurch gekennzeichnet, dass** das Polymer Poly(2-hydroxyethyl)-L-asparagin ist.

13. Konjugat nach Anspruch 1, **dadurch gekennzeichnet, dass** das Polymer Poly(D,L-methioninsulfoxid) ist.

## Revendications

1. Conjugué lipide-polymère, qui peut être obtenu d'un lipide amphiphile, constitué d'au moins un fragment apolaire hydrophobe et d'un groupe de tête polaire hydrophile, et d'un polymère ou d'un précurseur monomérique de celui-ci, ayant un groupe final en N et C-terminal, **caractérisé en ce que** le polymère est un acide polyaminé, un dérivé d'acide polyaminé ou un analogue d'acide polyaminé et **en ce que** le lipide est attaché au groupe final en N ou C-terminal du polymère, le polymère ayant la formule suivante:
- [NHCHR(CH₂)ₘCO]ₙ-
dans laquelle :
-R est défini en tant que:
-H, -CH₃, -CHCH₃OR₁, -(CH₂)ₓOR₁, -(CH₂)ₓ-CO-NHR₁, -(CH₂)ₓ-NH-CO-R₁, -(CH₂)ₓ-SO_{y}CH₃, ou -(CH₂)ₓCOOH ;
-R₁ est un atome H ou un groupe alkyle en (C₁ à C₄), substitué par un ou plusieurs groupes hydroxy ou un groupe di(alkyle en C₁ à C₄) amine ;
x est 0 à 4 ;
m = 1 ou 0 et
y = 1 ou 2.

2. Conjugué selon la revendication 1, **caractérisé en ce que** le lipide amphiphile est choisi dans le groupe constitué de phospholipides, glycolipides, céramides, cholestérol et dérivés, mono ou dialkylamines, arylalkylamines, cycloalkylalkylamines, alcanols, aldéhydes, carbohalogénures ou acides alcanoïques et leurs anhydrides en C₈ à C₅₀ à chaîne ramifiée ou linéaire, saturés ou insaturés, et **caractérisé en ce que** le nombre total d'atomes C est de 25 ou plus.

3. Conjugué selon la revendication 2, **caractérisé en ce que** le lipide amphiphile contient au moins deux fragments apolaires hydrophobes.

4. Conjugué selon la revendication 3, **caractérisé en ce que** le lipide amphiphile est choisi dans le groupe constitué de la 1-heptadécyloctadécylamine, la N-succinyl-dioctadécylamine et la distéarylphosphatidyléthanolamine.

5. Conjugué selon l'une quelconque des revendications précédentes, **caractérisé en ce que** le polymère à un paramètre χ ≤ 0,65, de préférence ≤ 0,5 dans l'eau.

6. Conjugué selon l'une quelconque des revendications précédentes, **caractérisé en ce que** le polymère est constitué d'acides α-aminés et de dérivés ou d'analogues de ceux-ci.

7. Conjugué selon l'une quelconque des revendications précédentes, **caractérisé en ce que** le polymère ne contient pas une quantité conséquente de groupes chargés dans un pH physiologique de 4 à 8.

8. Conjugué selon la revendication 7, **caractérisé en ce que** le polymère est constitué de monomères d'acide aminé, de monomères d'analogue d'acide aminé ou de monomères de dérivés d'acide aminé, qui sont neutres ou qui ont été neutralisés à un pH physiologique de 4 à 8.

9. Conjugué selon l'une quelconque des revendications précédentes, **caractérisé en ce que** le polymère a un poids moléculaire compris entre 500 et 75 000, de préférence entre 2 000 et 15 000.

10. Conjugué selon l'une quelconque des revendications précédentes, **caractérisé en ce que** le polymère est biodégradable.

11. Conjugué selon la revendication 1, **caractérisé en ce que** le polymère est la poly[N-(2-hydroxyéthyl)]-L-glutamine.

12. Conjugué selon la revendication 1, **caractérisé en ce que** le polymère est la poly(2-hydroxyéthyl)-L-asparagine.

13. Conjugué selon la revendication 1, **caractérisé en ce que** le polymère est le poly(sulfoxyde de D,L-méthionine).
